# EUROPÄISCHE PATENTSCHRIFT

(11) **EP 0 632 009 B1**
(45) Veröffentlichungstag und Bekanntmachung des Hinweises auf die Patenterteilung: **30.07.1997**
(21) Anmeldenummer: 94108359.4
(22) Anmeldetag: 31.05.1994
(51) Int. Cl.: C07C 53/50, C07C 51/493, C08F 14/26, C08F 114/26, C08F 214/26

(54) **Verfahren zur Rückgewinnung von fluorierten Carbonsäuren**
Process for the recuperation of fluorinated carboxylic acids
Procédé pour la récupération d'acide carboxyliques fluorés

(30) Priorität: 02.06.1993 DE 4318258; 29.01.1994 DE 4402694
(43) Veröffentlichungstag der Anmeldung: 04.01.1995
(73) Patentinhaber: Dyneon GmbH, 84504 Burgkirchen (DE)
(72) Erfinder: Obermeier, Reinhold, D-84453 Mühldorf (DE); Stefaniak, Gunter, Dr., D-84508 Burgkirchen (DE)

(56) Entgegenhaltungen:
- EP-A- 0 214 808
- US-A- 4 038 231
- US-A- 4 391 940
- PATENT ABSTRACTS OF JAPAN, unexamined applications, C Section, Band 14, Nr. 137, 15. MUrz 1990 THE PATENT OFFICE JAPANESE GOVERNMENT Seite 62 C 702; & JP-A-02009836 (NISSAN CHEM.)

## Beschreibung

Für die Polymerisation fluorierter Monomerer in wäßriger Dispersion werden fluorierte Emulgatoren eingesetzt, da diese keine telogene Aktivität aufweisen. Hauptsächlich werden gutlösliche Salze von Perfluorcarbonsäuren, insbesondere das Ammoniumsalz der Perfluoroctansäure, verwendet. Für diesen Zweck müssen die zugrundeliegenden Säuren eine hohe Reinheit aufweisen, da Verunreinigungen das Anspringen der Polymerisation be- oder gar verhindern können, Störungen im Polymerisationsablauf, beispielsweise durch Kettenübertragung, auftreten können oder ein Abbruch der Polymerisation erfolgen kann.

Diese Reinheitskriterien machen bisher die Wiedergewinnung der genannten Säuren sehr schwierig und außerordentlich aufwendig. Erschwerend wirkt hierbei, daß die Säuren bei der Rückgewinnung durch Auswaschung mit Laugen, beispielsweise aus der Abluft, ihre Tensidwirkung entfalten, indem sie einen schwer zu beherrschenden Schaum bilden. Häufig werden deshalb Schaumdämpfer eingesetzt, die eine Aufarbeitung der zugrundeliegenden Säuren weiter erschweren.

Versucht man aus derartigen Gemischen die Säuren - beispielsweise mit Schwefelsäure - freizusetzen und anschließend durch Destillation zu gewinnen, so scheitert dies schon daran, daß hierbei diese Säuren kristallisierende Hydrate bilden, die die Destillationsapparatur zusetzen können. Außerdem werden hierbei Destillationsrückstände erzeugt, die noch erhebliche Mengen an Fluor enthalten und deren Entsorgung sehr aufwendig ist.

Es wurde nun ein Verfahren gefunden, das die Verwertung der genannten fluorierten Carbonsäuren aus Gemischen erlaubt, in denen diese Säuren auch als Salze vorliegen können, das die genannten Nachteile nicht aufweist. Erfindungsgemäß wird hierzu die fluorierte Carbonsäure aus dem Ausgangsmaterial im wäßrigen Medium mit einer hinreichend starken Säure nötigenfalls freigesetzt und mit einem geeigneten Alkohol zu einem Ester umgesetzt, der destillativ abgetrennt wird. Falls eine Freisetzung der Carbonsäure aus ihrem Salz nicht erforderlich ist, genügt selbstverständlich eine katalytische Menge an Säure für die Veresterung.

Als Ausgangsmaterial sind feste und flüssige Produkte geeignet, die Salze der fluorierten Carbonsäuren enthalten. In der Praxis handelt es sich hierbei um die Ammonium- oder Natriumsalze, aber selbstverständlich sind analoge Salze wie Kaliumsalze nicht ausgeschlossen. Die Ausgangsmaterialien können neben den bereits genannten Schaumdämpfern auch noch andere anorganische oder organische Verunreinigungen enthalten, sofern diese nicht flüchtige Verbindungen bilden, die bei der Destillation des gewünschten Esters stören.

Als Ausgangsmaterial kann vorteilhaft eine Polymerisationsflotte eingesetzt werden. Das bevorzugte Ausgangsmaterial sind Flotten aus der Emulsionspolymerisation fluorierter Polymerer. Diese können unmittelbar nach Abtrennung des Polymers, also ohne Aufkonzentrierung, verwendet werden. Die Emulsionspolymerisation ist beispielsweise beschrieben in der US-A 4 391 940 (und der darin genannten früheren Literatur).

Als hinreichend starke Säure zur Freisetzung der fluorierten Carbonsäure verwendet man in der Praxis Schwefelsäure. Selbstverständlich sollen aber auch hier gleichwirkende Säuren nicht ausgeschlossen werden.

Als Alkoholkomponente kommt in erster Linie Methanol in Betracht, da es preiswert ist und einen leichtflüchtigen Ester bildet. Der Einsatz anderer Alkohole, beispielsweise Ethanol oder Isopropanol, ist möglich, erbringt aber keine Vorteile.

Im folgenden werden weitere vorteilhafte Ausgestaltungen der Erfindung näher erläutert, wobei der Einfachheit halber jeweils nur bevorzugte Reaktionsteilnehmer beziehungsweise Reagenzien erwähnt werden. Die Perfluoroctansäure wird hierbei als "PFOS" abgekürzt. Prozente sind Gewichtsprozente, wenn keine anderen Angaben gemacht sind.

Ein Destillationsgefäß wird mit wäßriger Schwefelsäure, vorzugsweise mit 60%iger Schwefelsäure, in einer solchen Menge beschickt, daß aus dem anschließend eingetragenen Ausgangsmaterial, beispielsweise dem Natriumsalz der PFOS - in Lösung, in fester Form oder als Suspension, die noch freie Natronlauge enthalten kann - die PFOS freigesetzt wird. Zweckmäßig enthält die genannte Suspension etwa 1 bis etwa 70 %, vorteilhaft etwa 5 bis etwa 40 %, des Natriumsalzes und nicht mehr als 40 % freie Natronlauge. Beim Einsatz einer Polymerisationsflotte liegt der Gehalt an PFOS-Salz wesentlich niedriger, beispielsweise bei 0,01 bis 0,1 %. Anschließend wird eine überschüssige Menge Methanol, zweckmäßig 10 mol pro mol PFOS, zudosiert und das Reaktionsgemisch langsam auf eine Sumpftemperatur von etwa 70 bis etwa 90 °C, vorzugsweise 85 °C, erhitzt. Ab dieser Temperatur destilliert ein ternäres Gemisch aus Wasser, Methanol und PFOS-Methylester über, das sich nach der Kondensation in zwei Phasen auftrennt. Die untere, schwerere Phase (Dichte 1,7 g/cm³) besteht aus über 98 % PFOS-Methylester und die obere Phase aus einem Gemisch von Methanol und Wasser. Die untere Phase wird abgetrennt und die obere Phase in den Prozeß zurückgeführt. Die Destillation wird solange fortgesetzt, bis im Destillat keine Phasentrennung mehr erfolgt. Das verbleibende Methanol- Wassergemisch wird abgetrennt, zweckmäßig abdestilliert, und für einen folgenden Ansatz wiederverwendet. Als Destillationsrückstand bleibt eine circa 25 % Natriumsulfat und circa 5 % Schwefelsäure enthaltende wäßrige Lösung, die nur etwa 0,1 % organische Substanz und nur etwa 0,01 % Fluor enthält.

Bei der bevorzugten Ausgestaltung der Erfindung unter Einsatz einer rohen Polymerisationsflotte setzt man dieser eine kleine Menge, beispielsweise 2 bis 5 %, an Alkohol, vorzugsweise Methanol, zu, stellt mit einer starken Säure, bevorzugt Schwefelsäure, die Flotte hinreichend sauer, vorzugsweise auf einen pH-Wert von etwa 1 bis 2, und destilliert den gebildeten Ester ab. Wenn die rohe Polymerisationsflotte noch geringe Mengen feinteiligen Polymerisats enthält, klärt sich hierbei die trübe Flotte auf, wobei das Polymerisat in leicht abtrennbarer Form anfällt. Durch die erfindungsgemäße Behandlung wird also die oberflächenaktive fluorierte Carbonsäure weitestgehend abgetrennt. Dies zeigt sich auch darin, daß der ursprüngliche Fluorgehalt der Flotte erheblich reduziert wird.

In einer besonders bevorzugten Ausgestaltung der Erfindung wird der PFOS-Methylester direkt in das Ammoniumsalz der PFOS überführt. Überraschenderweise kann hierzu der anfallende Rohester unmittelbar mit wäßriger Ammoniaklösung verseift werden, wodurch ein Produkt in der erforderlichen Reinheit gewonnen wird.

Vorteilhaft wird hierzu wäßrige Ammoniaklösung von zweckmäßig 25 % Ammoniakgehalt und Wasser in ein Rührgefäß gegeben und hierzu der Rohester zudosiert. Man erwärmt auf etwa 70 bis 95 °C und rührt solange, bis die anfangs trübe Reaktionsmischung vollständig klar wird. Bei einer Sumpftemperatur von etwa 95 °C wird dann ein Gemisch von Methanol und Wasser abdestilliert, das für die Veresterung eingesetzt werden kann. Die zurückbleibende Lösung des Ammoniumsalzes der PFOS kann - nötigenfalls nach Filtration - unmittelbar als Emulgator für die Polymerisation fluorierter Monomerer eingesetzt werden. Der Amidgehalt ist unter 0,1 %.

Insbesondere in der vorstehend genannten bevorzugten Ausgestaltung gelingt es also, in einem eleganten, unaufwendigen Verfahren den teuren Wertstoff PFOS-Ammoniumsalz in hoher Ausbeute und der geforderten Reinheit aus stark verunreinigten Ausgangsmaterialien wiederzugewinnen. Besonders vorteilhaft ist, daß bei diesem Verfahren keine schwer zu entsorgenden Abfälle erhalten werden, auch dann, wenn das eingesetzte Material Verunreinigungen wie Schaumdämpfer enthält.

Der anfallende PFOS-Methylester kann natürlich auch in anderer Weise weiterverarbeitet werden, beispielsweise zu freier PFOS verseift werden. Da ein Ester ein aktiviertes Säurederivat darstellt, sind selbstverständlich auch andere übliche Weiterverarbeitungsmaßnahmen möglich, wie Umesterungen oder Überführung in Amide.

Die Erfindung wird in den folgenden Beispielen näher erläutert. Sofern keine anderen Angaben gemacht werden, sind auch hier Prozente Gewichtsprozente und Teile Gewichtsteile.

### Beispiel 1

In einem Rührgefäß werden 2000 Teile 60%ige Schwefelsäure vorgelegt und auf 50 °C erwärmt. Unter Rühren werden dann 3000 Teile PFOS-Natriumsalz in Form einer Suspension zudosiert, die 15 % des Natriumsalzes und 25 % Natriumhydroxid enthält. Hierbei wird durch Kühlen die Temperatur bei etwa 60 °C gehalten. Anschließend werden 350 Teile Methanol langsam zudosiert und das Gemisch allmählich erhitzt. Ab einer Sumpftemperatur von 85 °C destilliert ein ternäres Gemisch aus Wasser, Methanol und PFOS-Methylester über. Das Destillat trennt sich nach der Kondensation in zwei Phasen auf: Die untere Phase (Dichte 1,7 g/cm³) besteht aus PFOS-Methylester mit einer Reinheit größer 98 %, die obere Phase aus 70%igem Methanol. Die obere Phase wird in das Destillationsgefäß zurückgeführt und das Verfahren solange fortgesetzt, bis im Kondensat keine Phasentrennung mehr erkennbar ist.

Das überschüssige Methanol wird als 70%iges Methanol-Wassergemisch abdestilliert und für einen folgenden Ansatz verwendet. Der Destillationsrückstand besteht aus Wasser mit einem Gesamtfluorgehalt von 0,01 %, einem Gehalt an Natriumsulfat von circa 25 %, einem Gehalt an Schwefelsäure von circa 5 % und einem Gehalt von etwa 0,1 % organischer Substanz entsprechend einem CSB-Wert von 1500 mg O₂/kg.

### Beispiel 2

Als Ausgangsmaterial dient eine Rohflotte aus der Copolymerisation von Tetrafluorethylen und Perfluor-(n-propyl-vinyl)ether, bei der das Ammoniumsalz der PFOS als Emulgator eingesetzt war. Der Gesamtfluorgehalt der Flotte beträgt 0,14 %.

In einem Rührgefäß werden 4000 g dieser Flotte mit 120 g Methanol und dann mit soviel 60%iger Schwefelsäure versetzt, daß sich ein pH-Wert von 1 einstellt. Das Gemisch wird zum Sieden erhitzt, so daß langsam ein Gemisch aus Methanol, PFOS-Methylester und Wasser abdestilliert. Hierbei wird die anfangs trübe Flotte vollständig klar, wobei sich ein weißer Niederschlag des Polymerisats bildet.

Das Destillat trennt sich nach der Kondensation in zwei Phasen auf: Die untere Phase (Dichte 1,7 g/cm³) besteht aus PFOS-Methylester, die obere Phase aus 70%igem Methanol. Dieses wird abgetrennt und in einem folgenden Ansatz verwendet. Das Verfahren wird solange fortgesetzt, bis im Kondensat keine Phasentrennung mehr erkennbar ist. Das klare Filtrat des Destillationsrückstands enthält etwa 10 ppm Fluor.

### Beispiel 3

Das Beispiel 2 wird mit einer Rohflotte wiederholt, die aus der Homopolymerisation von Tetrafluorethylen nach dem Emulsionsverfahren mit dem Ammoniumsalz der PFOS als Emulgator stammt. Die Rohflotte enthält einen Gesamtfluorgehalt von 0,02 %, nach der Behandlung von circa 10 ppm.

### Beispiel 4

In ein Destillationsgefäß werden 1504 Teile Wasser und 150 Teile 25%ige Ammoniaklösung gegeben und unter Rühren werden 428 Teile PFOS-Methylester langsam zudosiert. Das Gemisch wird zunächst auf 75 °C erhitzt, bei dieser Temperatur 30 Minuten gerührt und anschließend 5 Stunden bei 90 °C gerührt. Hierbei wird die anfangs trübe Mischung vollständig klar. Man erhitzt weiter bis zu etwa 95 °C Sumpftemperatur, wobei bei einer Kopftemperatur von 70 bis 99 °C 680 Teile eines etwa 5%igen Methanol-Wassergemisches überdestillieren. Als Destillationsrückstand bleibt eine wäßrige Lösung mit einem Gehalt an 30 % PFOS-Ammoniumsalz, die - nötigenfalls nach Filtrieren - unmittelbar als Emulgator für die Polymerisation fluorierter Monomerer eingesetzt werden kann.

## Patentansprüche

1. Verfahren zur Rückgewinnung von fluorierten Carbonsäuren in verwertbarer Form aus verunreinigten Ausgangsmaterialien, dadurch gekennzeichnet, daß man aus diesen Materialien im wäßrigen Medium mit einer hinreichend starken Säure die fluorierte Carbonsäure nötigenfalls freisetzt, diese mit einem geeigneten Alkohol umsetzt und den gebildeten Ester abdestilliert.

2. Verfahren nach Anspruch 1, dadurch gekennzeichnet, daß das Ausgangsmaterial Perfluoroctansäure oder ihr Salz enthält.

3. Verfahren nach Anspruch 1 oder 2, dadurch gekennzeichnet, daß das Ausgangsmaterial die fluorierte Carbonsäure als Ammonium- oder Natriumsalz enthält.

4. Verfahren nach einem oder mehreren der vorhergehenden Ansprüche, dadurch gekennzeichnet, daß das Ausgangsmaterial eine Polymerisationsflotte ist.

5. Verfahren nach einem oder mehreren der vorhergehenden Ansprüche, dadurch gekennzeichnet, daß der Alkohol Methanol ist.

6. Verfahren nach einem oder mehreren der vorhergehenden Ansprüche, dadurch gekennzeichnet, daß die hinreichend starke Säure Schwefelsäure ist.

7. Verfahren nach einem oder mehreren der vorhergehenden Ansprüche, dadurch gekennzeichnet, daß der gebildete Ester mit wäßriger Ammoniaklösung zum Ammoniumsalz der fluorierten Carbonsäure verseift wird.

## Claims

1. A process for the recovery of fluorinated carboxylic acids in utilizable form from contaminated starting materials, which comprises releasing, if necessary, the fluorinated carboxylic acid from these materials in an aqueous medium with a sufficiently strong acid, reacting this fluorinated carboxylic acid with a suitable alcohol and distilling off the ester formed.

2. The process as claimed in claim 1, wherein the starting material contains perfluorooctanoic acid or a salt thereof.

3. The process as claimed in claim 1 or 2, wherein the starting material contains the fluorinated carboxylic acid as ammonium salt or sodium salt.

4. The process as claimed in one or more of the preceding claims, wherein the starting material is a polymerization liquor.

5. The process as claimed in one or more of the preceding claims, wherein the alcohol is methanol.

6. The process as claimed in one or more of the preceding claims, wherein the sufficiently strong acid is sulfuric acid.

7. The process as claimed in one or more of the preceding claims, wherein the ester formed is hydrolyzed with aqueous ammonia solution to give the ammonium salt of the fluorinated carboxylic acid.

## Revendications

1. Procédé pour la récupération d'acides carboxyliques fluorés sous forme utilisable à partir de matières de départ souillées, caractérisé en ce que l'on libère, si nécessaire, à partir de ces matières en milieu aqueux, à l'aide d'un acide suffisamment fort, l'acide carboxylique fluoré, on le fait réagir avec un alcool approprié et on sépare par distillation l'ester formé.

2. Procédé selon la revendication 1, caractérisé en que la matière de départ contient l'acide perfluoroctanoïque ou son sel.

3. Procédé selon la revendication 1 ou 2, caractérisé en ce que la matière de départ contient l'acide carboxylique fluoré sous forme de sel de sodium ou d'ammonium.

4. Procédé selon l'une ou plusieurs des revendications précédentes, caractérisé en ce que la matière de départ est un bain de polymérisation.

5. Procédé selon l'une ou plusieurs des revendications précédentes, caractérisé en ce que l'alcool est le méthanol.

6. Procédé selon l'une ou plusieurs des revendications précédentes, caractérisé en ce que l'acide suffisamment fort est l'acide sulfurique.

7. Procédé selon l'une ou plusieurs des revendications précédentes, caractérisé en ce que l'on saponifie l'ester obtenu avec une solution d'ammoniac pour obtenir le sel d'ammonium de l'acide carboxylique fluoré.
